(19) 

**Europäisches Patentamt**
**European Patent Office**
**Office européen des brevets**

(11) **EP 1 814 329 A2**

(12) **EUROPEAN PATENT APPLICATION**

(43) Date of publication:
**01.08.2007 Bulletin 2007/31**

(51) Int Cl.:
*H04N 7/01* (2006.01)     *G06T 3/40* (2006.01)

(21) Application number: **06026410.8**

(22) Date of filing: **20.12.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **21.12.2005 KR 20050126746**

(71) Applicant: **MEDISON CO., LTD.**
**Kangwon-do 250-870 (KR)**

(72) Inventors:
• **Moon, Joo Hee**
**Samseong Jungang Heights**
**Gangnam-gu**
**Seoul 135-507 (KR)**

• **Song, Jae Eun**
**Seoul 131-822 (KR)**
• **Kim, Hye Jung**
**Gangnam-gu**
**Seoul 135-280 (KR)**
• **Song, Young Seuk**
**Gangnam-gu**
**Seoul 135-280 (KR)**

(74) Representative: **Schmid, Wolfgang**
**Lorenz & Kollegen**
**Patent- und Rechtsanwaltskanzlei**
**Alte Ulmer Strasse 2**
**D-89522 Heidenheim (DE)**

(54) **Method of forming an image using block matching and motion compensated interpolation**

(57) Embodiments of the present invention may provide a method of forming an image by using block matching algorithm and motion compensating interpolation, said method comprising: a) receiving neighboring first and second frames, each frame being divided into a plurality of blocks; b) checking whether to form an interpolation frame to be inserted between the first and second frames based on a correlation between the first and second frames; c) if it is determined to form the interpolation frame, determining a first motion vector between each block in the interpolation frame and each block in the first frame, and determining a second motion vector between each of the blocks in the interpolation frame and each of the blocks in the second frame; d) determining a motion vector of each block in the interpolation frame based on the first and second motion vectors; e) reconstructing the interpolation frame by applying the motion vector of each block, wherein pixel values of the interpolation frame are determined based on pixel values of the first and second frames; and f) if brokenness of the interpolation frame is less than a threshold, adopting the interpolation frame.

FIG. 2

(dx, dy) : MOTION VECTOR

(x1, y1) : POSITION FOR MCI

B(x1, y1) : IMAGE SIGNAL AT (x1, y1)

**Description**

The present application claims priority from Korean Patent Application No. 10-2005-0126746 filed on December 21, 2005, the entire subject matter of which is incorporated herein by reference.

**BACKGROUND**

**1. Field**

Embodiments of the present invention may generally relate to an image forming method, and more particularly to a method of forming an image using block matching algorithm and motion compensated interpolation.

**2. Background**

An ultrasound diagnostic system has become an important and popular diagnostic tool since it has a wide range of applications. Specifically, due to its non-invasive and non-destructive nature, the ultrasound diagnostic system has been extensively used in the medical profession. Modern high-performance ultrasound diagnostic systems and techniques are commonly used to produce two or three-dimensional diagnostic images of internal features of an object (e.g., human organs).

The ultrasound diagnostic system generally uses a wide bandwidth transducer to transmit and receive ultrasound signals. The ultrasound diagnostic system forms images of human internal tissues by electrically exciting an acoustic transducer element or an array of acoustic transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which are amplified and processed to produce ultrasound data for an image of the tissues. The ultrasound diagnostic system is very important in the medical field since it provides physicians with real-time and high-resolution images of human internal features without the need for invasive observation techniques such as surgery.

An ultrasound moving image has been long researched and developed for diagnosis and application purposes. Also, the use of the ultrasound moving image with the Internet and a mobile communication network is expected to proper in the near future. Therefore, it is important to acquire a reliable ultrasound moving image.

The moving image is implemented by consecutively displaying still images sequentially acquired during a short time (i.e., frame images). Generally, a change in brightness between frames may occur due to the movement of a target object or a camera. The change in brightness may be used to estimate the motion of the target object between neighboring frames. Motion estimation is extensively used to encode moving images and still images are advantageous in reducing an estimation error by searching motion information in a pixel unit. However, it is disadvantageous in that the calculating process is very complex. Therefore, it is extremely difficult to apply the motion estimation to a low bit rate image transmission system.

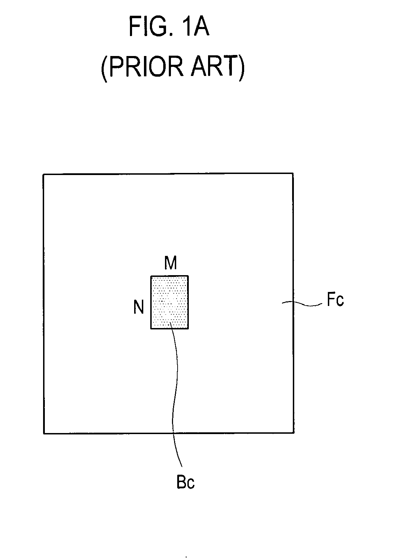

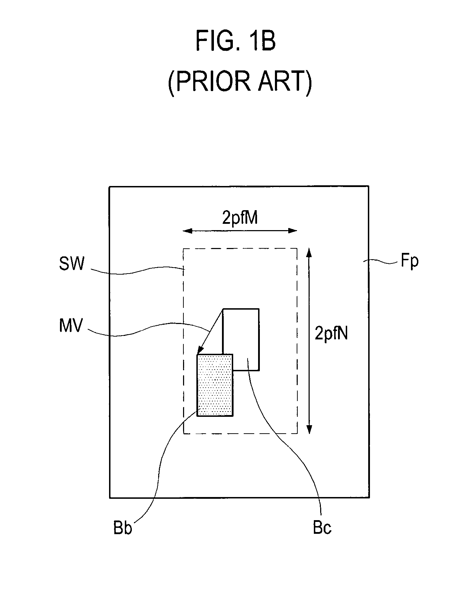

Recently, a block matching algorithm (BMA) has been adopted in order to reduce the amount of calculation for the motion estimation. According to BMA, a frame is divided into various regular sized blocks. Each block of a current frame Fc is matched to a block in a previous frame image by shifting the current block Bc over the previous frame image. Then, a best-matched block, which is closest to the current block Bc, is searched within a search window SW set on the previous frame Fp. Thereafter, the displacement between the best-matched block and the current block Bc is represented as a motion vector MV, as shown in Figs. 1A and 1B. Assuming that maximum horizontal and vertical displacements for the block of an M x N size are p pixels, the size of the search window can be selected as (2p+M) x (2p+N). The match between the current block Bc and the arbitrary block in the search window SW is defined by using a sum of the absolute difference (SAD), which is defined by the following equation.

$$SAD_{M \times N}(x, y) = \sum_{i=1, j=1}^{M,N} \left| Pc_{(x-i, y-j)} - Pp_{(x-i-dx, y-j-dy)} \right| \qquad (1)$$

Wherein, Pc is the intensity of each pixel comprising the current block Bc, Pp is the intensity of each pixel comprising the arbitrary block in the search window SW, x and y represent the coordinates of a specific pixel in the current block BC, and dx and dy represent the displacements between the current block Bc and the arbitrary block. The motion vector between the current block Bc in the current frame Fc and the arbitrary block Bb in the previous frame Fp, which is the best-matched block with the current block Bc, is determined by calculating the coordinate movement of the block resulting in a minimum SAD.

If the motion vector is incorrectly calculated according to the conventional BMA, then blocking artifacts such as a hole, an overlapped region and the like may occur. Therefore, the subjective image quality of images reconstructed between the current frame Fc and the previous frame Fp may become degraded.

In order to solve the above problem, a full search method of comparing the current block in the current frame Fc with the overall blocks existing in the search window is widely used to accurately obtain motion information. However, if the full search method is adopted to estimate motion, then the amount of computation increases. Therefore, it is difficult to form a moving image in real time.

## BRIEF DESCRIPTION OF THE DRAWINGS

Arrangements and embodiments may be described in detail with reference to the following drawings in which like reference numerals refer to like elements and wherein:

FIGS. 1A and 1B are schematic diagrams showing examples of determining a motion vector by using a conventional block matching algorithm.

FIG. 2 is a schematic diagram showing an example of forming a frame inserted between consecutive frames based on bidirectional motion estimation.

FIG. 3A and 3B are schematic diagram showing search windows set in consecutive frames for bidirectional motion estimation.

FIG. 4 is schematic diagram showing an example of a matching search technique.

FIGS. 5 to 7 are diagrams illustrating examples of classifying blocks in an interpolation frame formed based on a motion estimation vector and examining the brokenness of the blocks.

FIG. 8 is a diagram showing an example of block boundary reducing filtering.

## DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

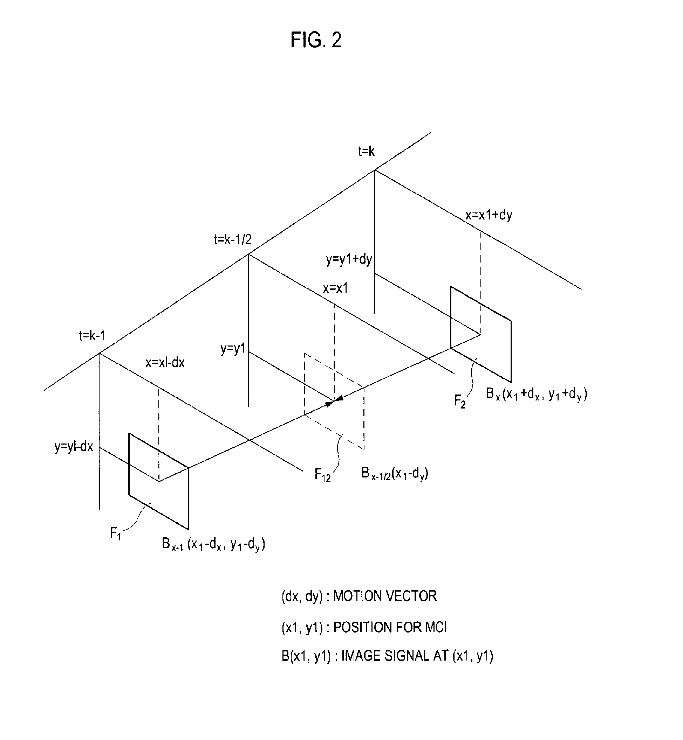

Fig. 2 is a schematic diagram for explaining a bidirectional interpolation method of forming frames to be inserted between consecutive frames $F_1$ and $F_2$ based on bidirectional motion estimation in accordance with one embodiment of the present invention. An interpolation frame $F_{12}$, which corresponds to a time of (k-1)/2, is reconstructed by using frames acquired at times of k-1 and k. As shown in Fig. 2, an image signal $B_{k-1/2}(x_1, y_1)$ of a reference pixel $(x_1, y_1)$ in the interpolation frame $F_{12}$ is produced by using an image signal $B_{k-1}(x_1 - d_x, y_1 - d_y)$ of a pixel $(x_1-d_x, y_1-d_y)$ in the frame $F_1$ and an image signal $B_k(x_1 + d_x, y_1 + d_y)$ of a pixel $(x_1+d_x, y_1+d_y)$. dx and dy represent the motion vectors. A motion vector is determined by estimating the bidirectional motion of a position of the pixel $(x_1, y_1)$ so as to produce the image signal $B_{k-1/2}(x_1, y_1)$.

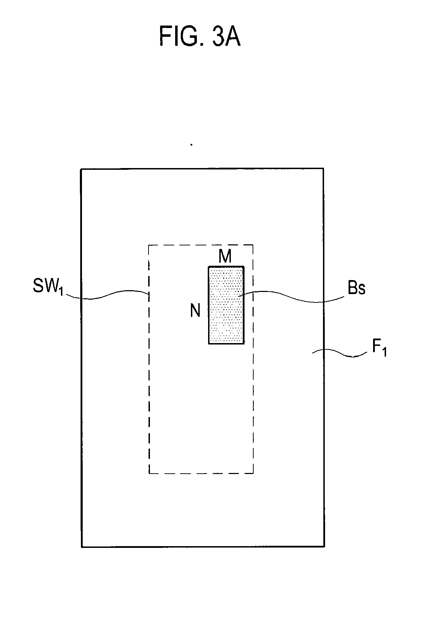

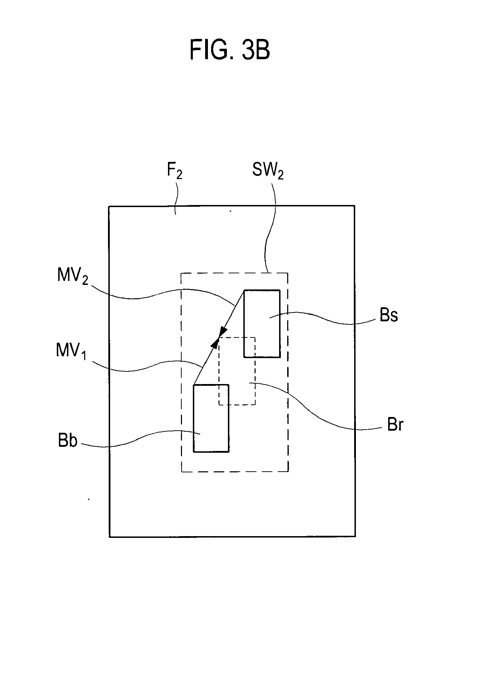

Search windows SW are respectively set in the frames F1 and F2 in an identical size for estimating the bidirectional motion, as shown in Figs. 3A and 3B. The search windows may be set with reference to a reference block Br existing in the frame $F_{12}$ to be interpolated. A selection block Bs is set in the Frame $F_1$. Further, a first motion vector $MV_1$ is determined based on the median coordinates of the reference block Br and the selection block Bs. A matching block Bm, which is best-matched with the selection block Bs, is determined in the frame F2. A second motion vector $MV_2$ is determined based on the median coordinates of the reference block Br and the matching block Bm.

For example, if a center of the reference block Br corresponds to an origin, then the pixels of the selection block Bs and the pixels of the matching block Bm are symmetric with respect to the origin. That is, the selection block Bs is symmetric with the matching block Bm with respect to the interpolation frame $F_{12}$. Therefore, a pixel positioned at $(x+v_3, y+v_3)$ in the frame $F_2$ corresponds to a pixel positioned at $(x-v_3, y-v_3)$ in the frame $F_1$. The motion vectors of each block in the interpolation frame $F_{12}$ are determined by applying a first motion vector $MV_1$ of $(x_1-d_x, y_1-d_y)$ and a second motion vector $MV_2$ of $(x_1 + d_x, y_1 + d_y)$ to each block. The interpolation frame $F_{12}$ is formed based on the determined motion vectors of each block.

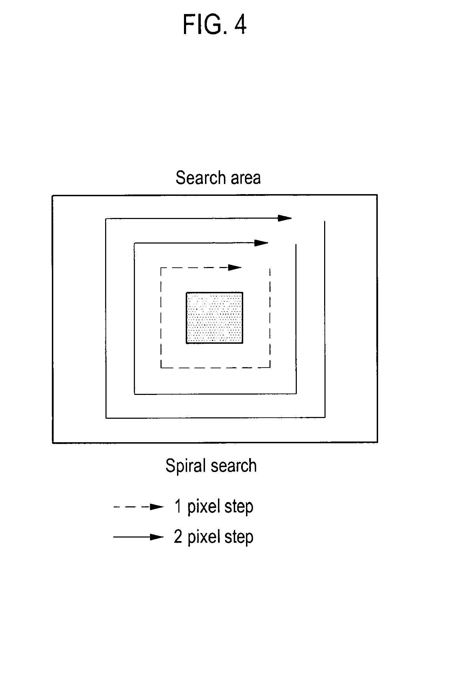

In order to more efficiently estimate the motion, the matching block is spirally searched, as shown in Fig. 4. A search interval may be changed based on the quantity of the motion vector. For example, if the first motion vector $MV_1$ is (0, 0), then the matching block Bm is searched in a one-pixel interval around the block. If something else, then the matching block Bm is searched in a two-pixel interval. If the matching block is searched in a two-pixel interval, then a spiral search is carried out by one pixel with reference to the searched matching block. This searching method may considerably increase the speed as well as possessing a similar efficiency as the full search method.

The determined motion vector of each block in the interpolation frame $F_{12}$ is smoothened through using a vector median filtering method. A typical median filtering method is implemented through using scalar filtering, which classifies the motion vector into components in horizontal and vertical directions. However, there is a problem in that the motion vector obtained by filtering the classified components may be expressed differently from neighboring motion vectors. In order solve the above problem, the filtering is carried out for the overall components of the motion vector. This vector median filtering is defined as follows:

$$\sum_i (| M_x(i) - M_x(med) | + | M_y(i) - M_y(med) |)$$

$$\leq \sum_i (| M_x(i) - M_x(j) | + | M_y(i) - M_y(j) |) \textit{ for any } j \qquad (2)$$

Further, in order to prevent the degradation of image quality in accordance with one embodiment of the present invention, it is determined whether the interpolation frame should be adopted according to the following process. After calculating SAD between the blocks in the interpolation frame obtained based on the motion vectors and the blocks in the current frame, the blocks in the interpolation frame are classified with reference to SAD. If SAD is relatively very small, then it may be interpreted that motion hardly occurs in the current block. Also, if the SAD is over a threshold, then the block may be a block obtained by an incorrectly estimated motion vector or the frames have rarely correlation between them. If the number of the blocks classified as blocks having SAD over the threshold in one frame is over a predetermined number, then it may be determined that the timely consecutive two frames may be images, which do not have correlation fully or partially. In such a case, an image quality may be degraded. Therefore, these frames are not adopted such that a natural image may be obtained.

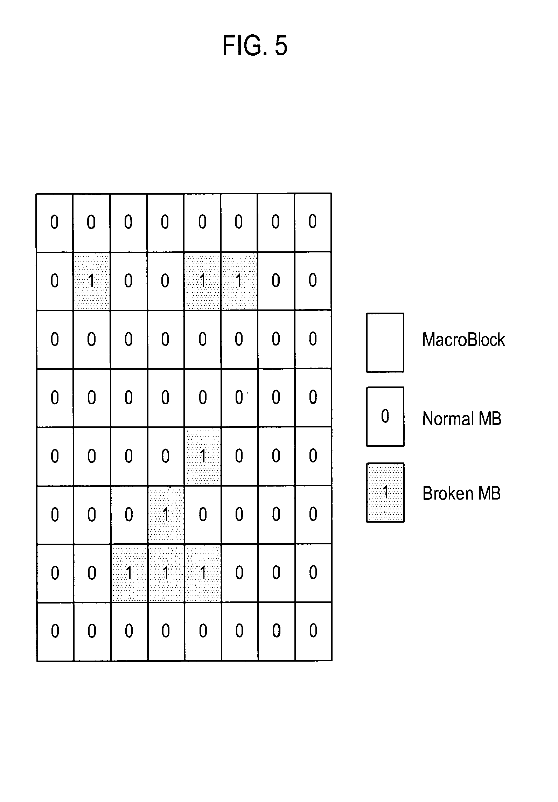

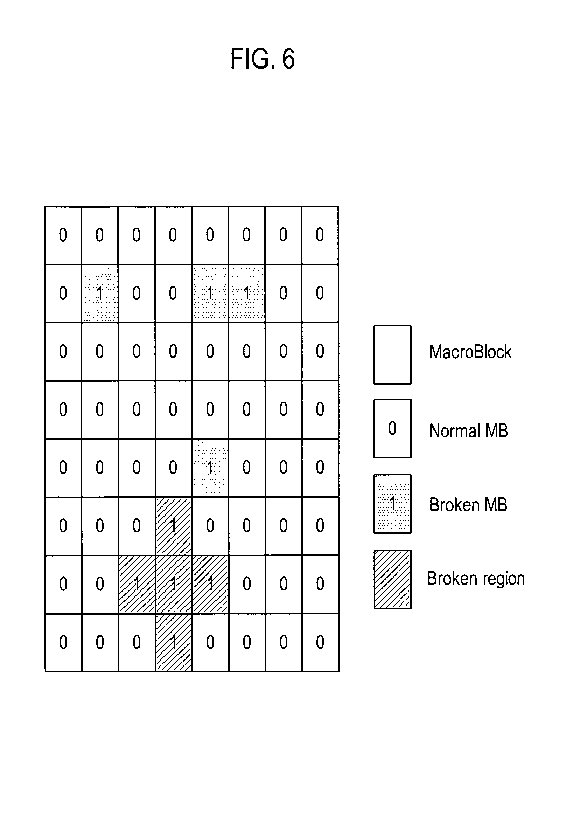

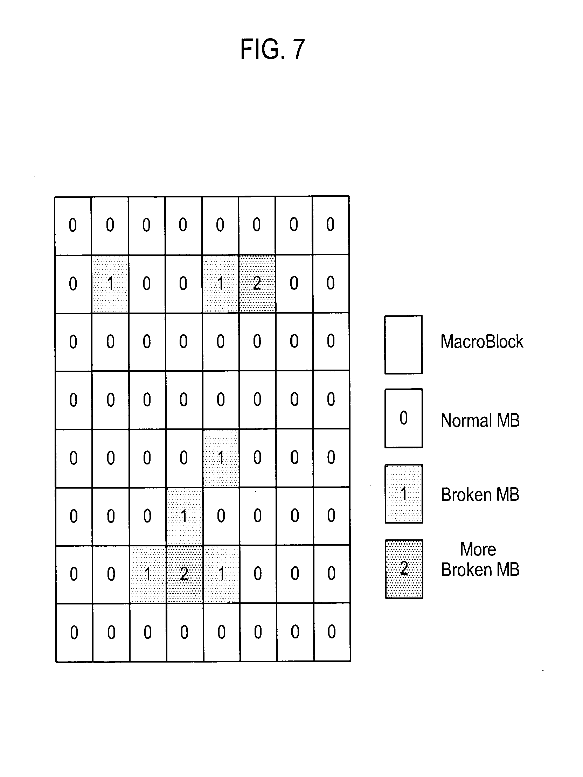

Figs. 5 to 7 show examples of examining brokenness of each block classified by SAD in the interpolation frames, which are formed based on the motion estimation vector. It is important to appropriately set a threshold for obtaining accurate interpolation frames. The brokenness for the overall blocks of the frame may be identified by using brokenness information classified into levels 1 and 2 and SAD information of each block, as shown in Figs. 5 to 7. By doing so, it can be determined whether to adopt the frame as the interpolation frame. A broken region in which broken blocks are concentrated may exist, as shown in Fig. 6. The blocks, which exist in up, down, right and left sides with reference to a specific block, are examined. Thereafter, if it is determined that the overall blocks correspond to the broken block, then the corresponding region may be designated to a broken region. Since the broken region causes image degradation, it may be determined whether to adopt the interpolation frame according to the size of a broken region and the number of the broken regions. Also, the broken region may be recovered by using deblocking filtering. Fig. 7 shows an example of classifying the blocks into the levels 1 and 2 based on the brokenness of the broken blocks. That is, if the brokenness of the block is relatively high, then it is determined that the block has no correlation with the neighboring blocks. In such a case, it is preferable to perform linear interpolation for the block so as to efficiently increase the image quality.

In case of forming only one interpolation frame, the interpolation frame may be formed through using the bidirectional interpolation method mentioned above. However, in case of forming a plurality of interpolation frames, the plurality of interpolation frames may be formed through using a weighted interpolation method defined as the following equation.

$$f(x, y, t_{1/2}) = \frac{l_2}{l_1 + l_2} f(x, y, t_0) + \frac{l_1}{l_1 + l_2} f(x, y, t_1) \qquad (3)$$

After determining whether to adopt the interpolation frames, deblocking filtering is carried out for smoothing any mismatches between the blocks. The blocking artifacts, which occur due to block mismatch, could be mostly smoothened by smoothing the motion vectors. However, block boundaries may be cleared due to differences in motion vectors between the blocks in the interpolation frame constructed in a block unit. Specially, when a correlation coefficient between the blocks within the search window in estimating the motion is relatively small, the block boundaries may be increasingly cleared. Therefore, in order to solve such problem, filtering is carried out for boundary pixels. However, if the boundary filtering is carried out for the overall block boundaries, then the processing time can be increased and the image blurring may also occur. Therefore, the blocks applying the boundary filtering are selected based on the motion vector information of the interpolation frame or SAD information. Then, the boundary filtering is carried out only for the selected blocks.

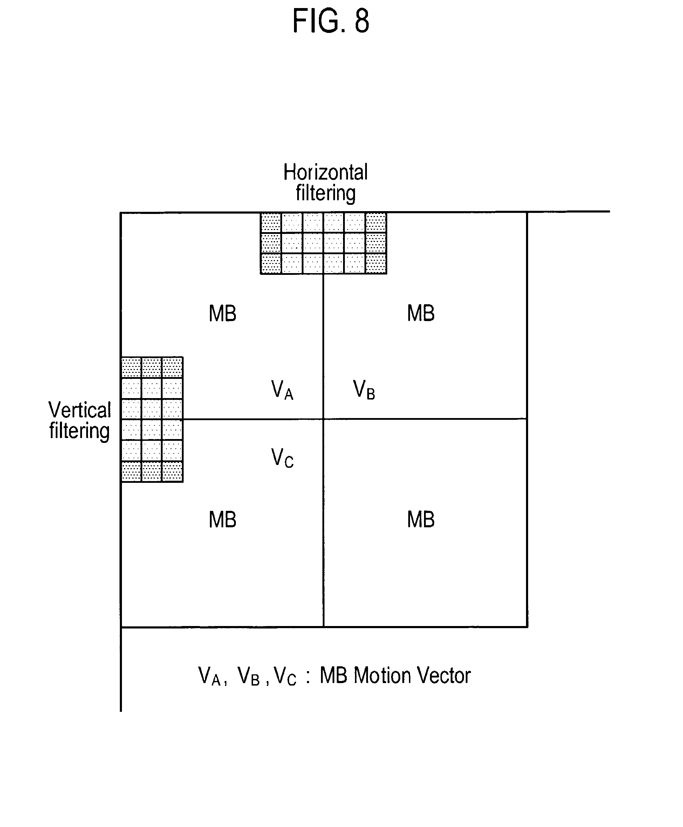

Fig. 8 is a diagram showing an example of a block boundary reducing filtering. As shown in Fig. 8, two pixels are selected at each boundary in horizontal and vertical directions, respectively. Then, the boundary filtering is carried out for the selected pixels.

The motion vectors of neighboring blocks in the interpolation frame are compared with each other. If the difference between the motion vectors is greater than a threshold, then the boundary filtering is carried out for the overall boundaries. On the other hand, if something else, then the next blocks are compared. That is, the norm of the motion vectors of the neighboring blocks is calculated. If the norm is greater than the threshold, then the boundary filtering may be carried out. The comparison of the motion vectors of the neighboring blocks may be expressed as the following equation.

$$\left\| V_A - V_B \right\| \geq T_1 \text{ or } \left\| V_A - V_C \right\| \geq T_1 \qquad (4)$$

In the equation (4), $V_A$ and $V_B$ represent motion vectors of neighboring blocks in a horizontal direction, while $V_A$ and $V_C$ represent motion vectors of neighboring blocks in a vertical direction. T1 represents a threshold.

The blocks to be filtered may be selected by using SAD of each block existing in the interpolation frame according to the above process. Also, when it is satisfied that the difference in the motion vectors of neighboring blocks is greater than the threshold and SAD of each block is greater than the threshold, the blocks are selected to be filtered.

If the blocks to be filtered are neighbored in a horizontal direction, then a filtering mask is masked to each pixel in a horizontal direction. Also, if the blocks to be filtered are neighbored in a vertical direction, then a filtering mask is masked to each pixel in a vertical direction. The pixel values obtained through filtering are replaced with the original pixels of the blocks.

As mentioned above, the motion vectors are determined by using bidirectional motion estimation with reference to the interpolation frame in order to reduce the blocking artifacts such as the hole region and the overlapped region occurring in a motion estimation process using the block matching algorithm. Thus, the motion compensating interpolation is more easily carried out, thereby forming the interpolation frame. Also, since the two-step searching method is employed, it has an effect similar to the full region searching method and the searching speed is considerably improved. Further, since it is determined whether to adopt the interpolation frame in consideration of correlation with a neighboring original frame, the efficiency is improved.

A method of forming an image, comprises: a) receiving neighboring first and second frames, each frame containing pixels divided into a plurality of blocks; b) determining whether to form an interpolation frame containing pixels divided into a plurality of blocks between the first and second frames based on a correlation between the first frame and the second frame; c) selecting one of blocks from the interpolation frame as a reference block; selecting a first block corresponding to the reference block from the first frame; determining a first motion vector between the reference block and the first block; selecting a second block corresponding to the reference block from the second frame and determining a second motion vector between the reference block and the second block; d) determining motion vectors of each block in the interpolation frame based on the first and second motion vectors; e) forming the interpolation frame by applying the motion vectors of each block and determining pixel values based on the first and second frames; and f) if brokenness of the interpolation frame is less than a threshold, then adopting the interpolation frame and forming an image.

The embodiment of the present invention provides a method of forming an image by using the block matching technique and a motion compensating interpolation capable of reducing an amount of calculation and having an exact estimation efficiency such as the full search method. The image may be a moving image, an ultrasound image and an ultrasound moving image.

Any reference in this specification to "one embodiment," "an embodiment," "example embodiment," etc., means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1. A method of forming an image, comprising:

   a) receiving neighboring first and second frames, each frame being divided into a plurality of blocks;
   b) checking whether to form an interpolation frame to be inserted between the first and second frames based on a correlation between the first and second frames;
   c) if it is determined to form the interpolation frame, determining a first motion vector between each block in the interpolation frame and each block in the first frame, and determining a second motion vector between each of the blocks in the interpolation frame and each of the blocks in the second frame;
   d) determining a motion vector of each block in the interpolation frame based on the first and second motion vectors;
   e) reconstructing the interpolation frame by applying the motion vector of each block, wherein pixel values of the interpolation frame are determined based on pixel values of the first and second frames; and
   f) if brokenness of the interpolation frame is less than a threshold, adopting the interpolation frame.

2. The method of Claim 1, wherein the step b) includes:

   b1) comparing the correlation with a threshold;
   b2) if the correlation is less than the threshold, returning to the step a); and
   b3) if the correlation is greater than the threshold, determining on forming the interpolation frame.

3. The method of Claim 1, wherein the step c) includes:

   c1) setting search windows in an identical size in the first and second frames with reference to a reference block in the interpolation frame;
   c2) selecting a first block from the first frame;
   c3) determining the first motion vector based on median coordinates of the reference block and median coordinates of the first block;
   c4) determining the second block matched with the first block in the second frame; and
   c5) determining the second motion vector based on median coordinates of the reference block and median coordinates of the second block.

4. The method of Claim 3, wherein in the step c4), the second block is determined by searching matching with a change of a pixel interval according to a quantity of the first motion vector.

5. The method of Claim 3, wherein the step c4) includes:

   c41) if the quantity of the first motion vector is equal to a threshold, searching a block matching the first block by spirally moving from a center of the search window by a first pixel interval;
   c42) if the quantity of the first motion vector is greater than the threshold, searching a block matching the first block by spirally moving from a center of the search window by a second pixel interval longer than the first pixel interval; and
   c43) if the block matching the first block is searched at the step c42), determining the second block by searching a block matching the first block by moving from the matched block by a third pixel interval shorter than the second pixel interval.

6. The method of Claim 1, after the step c), further comprising smoothening the first and second motion vectors through using vector median filtering.

7. The method of Claim 1, wherein in the step e), a plurality of interpolation frames are formed through an interpolation technique using weighted information.

8. The method of Claim 1, wherein the step f) includes:

f1) calculating a sum of absolute difference (SAD) between blocks in the interpolation frame; and
f2) if the number of blocks having SAD greater than a threshold is less than a reference number, adopting the interpolation frame.

9. The method of Claim 8, further comprising:

g) identifying whether each block in the interpolation frame is broken based on SAD; and
h) recovering broken blocks.

10. The method of Claim 9, further comprising:

g1) determining blocks to filter boundaries thereof; and
g2) filtering the determined blocks..

11. The method of Claim 10, wherein the step g1) includes:

g11) calculating a difference between motion vectors of neighboring blocks in the interpolation frame; and
g12) determining the blocks to filter boundaries thereof based on the calculated difference between the motion vectors.

12. The method of Claim 10, wherein the blocks to be filtered are determined based on SAD of the blocks in the interpolation frame at step g1).

13. The method of Claim 11, wherein the blocks to be filtered are determined based on the calculated difference between the motion vectors and SAD.

# FIG. 1A
# (PRIOR ART)

M

N

Fc

Bc

# FIG. 1B
# (PRIOR ART)

# FIG. 2

t=k

x=x1+dy

t=k-1/2

y=y1+dy

x=x1

t=k-1

x=xl-dx

y=y1

$B_x(x_1+d_x,\ y_1+d_y)$

$F_2$

y=yl-dx

$F_{12}$

$B_{x-1/2}(x_1-d_y)$

$F_1$

$B_{x-1}(x_1-d_x,\ y_1-d_y)$

(dx, dy) : MOTION VECTOR

(x1, y1) : POSITION FOR MCI

B(x1, y1) : IMAGE SIGNAL AT (x1, y1)

# FIG. 3A

# FIG. 3B

# FIG. 4

Search area

Spiral search

- - - ► 1 pixel step

——► 2 pixel step

# FIG. 5

| 0 | 0 | 0 | 0 | 0 | 0 | 0 | 0 |
|---|---|---|---|---|---|---|---|
| 0 | 1 | 0 | 0 | 1 | 1 | 0 | 0 |
| 0 | 0 | 0 | 0 | 0 | 0 | 0 | 0 |
| 0 | 0 | 0 | 0 | 0 | 0 | 0 | 0 |
| 0 | 0 | 0 | 0 | 1 | 0 | 0 | 0 |
| 0 | 0 | 0 | 1 | 0 | 0 | 0 | 0 |
| 0 | 0 | 1 | 1 | 1 | 0 | 0 | 0 |
| 0 | 0 | 0 | 0 | 0 | 0 | 0 | 0 |

☐ MacroBlock

|0| Normal MB

|1| Broken MB

# FIG. 6

| 0 | 0 | 0 | 0 | 0 | 0 | 0 | 0 |
|---|---|---|---|---|---|---|---|
| 0 | 1 | 0 | 0 | 1 | 1 | 0 | 0 |
| 0 | 0 | 0 | 0 | 0 | 0 | 0 | 0 |
| 0 | 0 | 0 | 0 | 0 | 0 | 0 | 0 |
| 0 | 0 | 0 | 0 | 1 | 0 | 0 | 0 |
| 0 | 0 | 0 | 1 | 0 | 0 | 0 | 0 |
| 0 | 0 | 1 | 1 | 1 | 0 | 0 | 0 |
| 0 | 0 | 0 | 1 | 0 | 0 | 0 | 0 |

☐ MacroBlock

0  Normal MB

1  Broken MB

▨  Broken region

# FIG. 7

# FIG. 8

Horizontal
filtering

Vertical
filtering

| MB | MB |
| $V_A$ | $V_B$ |
| $V_C$ | |
| MB | MB |

$V_A$, $V_B$, $V_C$ : MB Motion Vector

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020050126746